# EUROPEAN PATENT SPECIFICATION

(11) **EP 4 093 262 B1**
(45) Date of publication and mention of the grant of the patent: **10.01.2024**
(21) Application number: 21701185.7
(22) Date of filing: 22.01.2021
(51) Int. Cl.: A61B 3/00, A61B 3/024, A61B 3/113

(54) **COMPACT EXAMINATION APPARATUS WITH VISUAL FIELD PERIMETRY AND BLIND SPOT CAPABILITIES**
KOMPAKTES UNTERSUCHUNGSGERÄT MIT GESICHTSFELDPERIMETRIE- UND SKOTOMFÄHIGKEIT
APPAREIL D'EXAMEN COMPACT PRÉSENTANT DES CAPACITÉS DE PÉRIMÉTRIE DE CHAMP VISUEL ET DE TACHE AVEUGLE

(30) Priority: 23.01.2020 IT 202000001255
(43) Date of publication of application: 30.11.2022
(73) Proprietor: NIDEK CO. LTD., Gamagori, Aichi 443-0038 (JP)
(72) Inventor: PASCOLINI, Michele, 35128 Padova (IT); TANASSI, Cesare, 31053 Pieve di Soligo (TV) (IT); CARRARO, Federico, 35010 Villanova di Camposampiero (PD) (IT); CODOGNO, Nicola, 35020 Albignasego (PD) (IT); DE GIUSTI, Andrea, 35128 Padova (IT); MINOZZI, Mattia, 35020 Legnaro (PD) (IT); PAJARO, Ronnye, 30010 Campagna Lupia (VE) (IT); PETTINELLO, Cristiano, 35040 Villa Estense (PD) (IT); SCATTOLIN, Nikita, 30038 Spinea (VE) (IT)
(74) Representative: De Ros, Alberto
(86) International application number: PCT/IB2021/050505
(87) International publication number: WO 2021/149006

(56) References cited:
- WO-A1-97/35223
- DE-A1-102015 217 682
- FR-A1- 3 065 832
- US-A1- 2018 020 910

## Description

### TECHNICAL FIELD

The present invention relates to a compact examination apparatus with visual field perimetry and blind spot capabilities.

### BACKGROUND

Visual field perimetry testing has been carried out for several decades.

The well-known professional apparatuses for such test are quite bulky and are arrangements of sparse components.

More recently, compact apparatuses have been made available; for example, patent document DE 10 2015 217 682 A1 discloses such an apparatus.

### SUMMARY

The general purpose of the present invention is an examination apparatus with visual field perimetry and blind spot capabilities being compact, relatively simple, and providing accurate examination results.

According to a first more specific purpose of the present invention, the examination apparatus should be head-mounted.

According to a second more specific purpose of the present invention, the examination apparatus should have a simple optical assembly, i.e. including few lenses.

According to a third more specific purpose of the present invention, the examination apparatus should be simple in the sense of avoiding any auto-focus capabilities.

Such purposes are achieved through the examination apparatus having the features set out in the appended claims that have to be considered an integral part of the present description.

### BRIEF DESCRIPTION OF THE DRAWINGS

A more complete appreciation of the disclosed embodiment of the present invention and many of the attendant advantages thereof may be understood by referring to the following detailed description and by considering the accompanying drawings, wherein:
Fig. 1 is a (simplified) lateral cross-section view of an embodiment of an examination apparatus associated to a patient;
Fig. 2 is a (simplified) top cross-section view of the embodiment of Fig. 1 associated to a patient;
Fig.3A is a simplified and partial lateral cross-section view of the embodiment of Fig. 1 associated to a patient;
Fig.3B is a simplified and partial top cross-section view of the embodiment of Fig. 1 associated to a patient;
Fig. 4 is a first perspective view (partially sectioned) of the embodiment of Fig. 1 associated to a patient;
Fig. 5 is a second perspective view (partially sectioned) of the embodiment of Fig. 1 associated to a patient - it is to be noted that in this figure a shell of the examination apparatus is sectioned while an integrated assembly of the examination apparatus is not sectioned;
Fig. 6 is a third perspective view of the embodiment of Fig. 1; and
Fig. 7 is a perspective section view of an integrated assembly of the embodiment of Fig. 1.

It is to be noted that the present invention is not restricted to what described in the following, but its scope is determined solely by the appended claims.

### DETAILED DESCRIPTION OF EMBODIMENTS

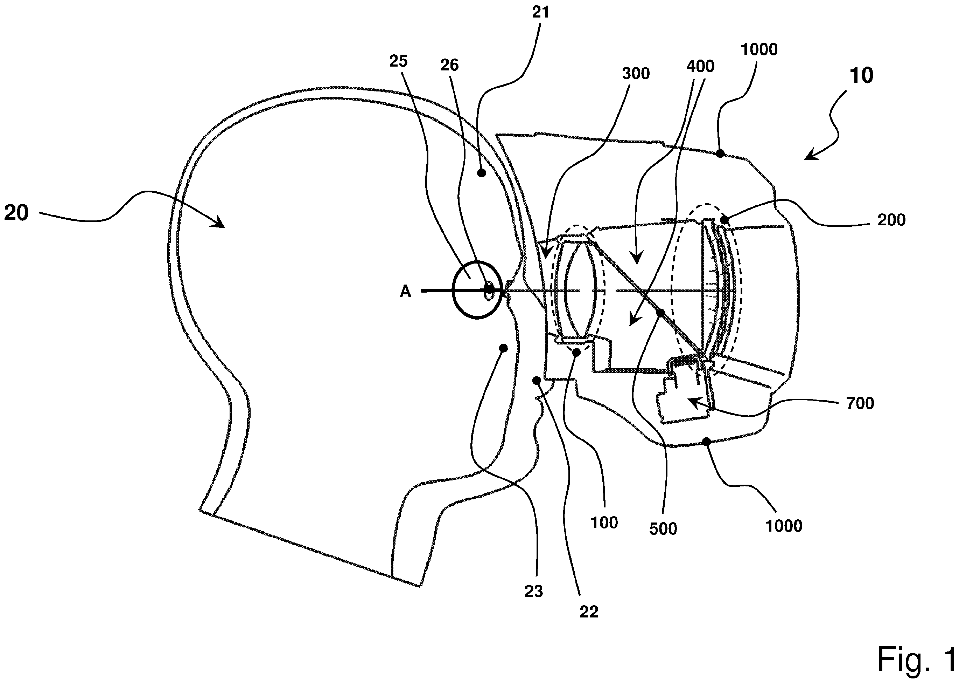

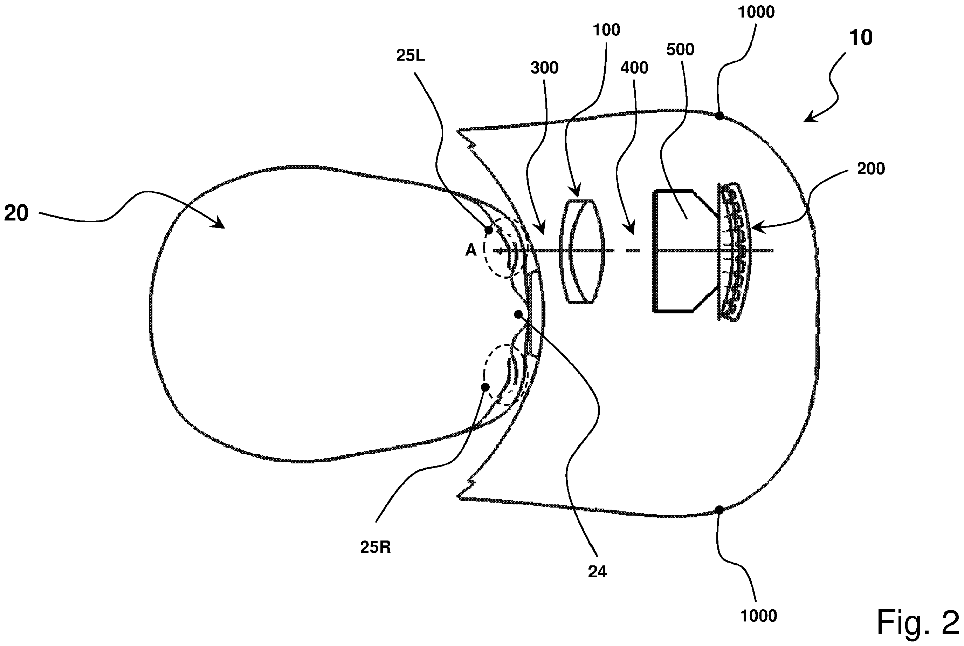

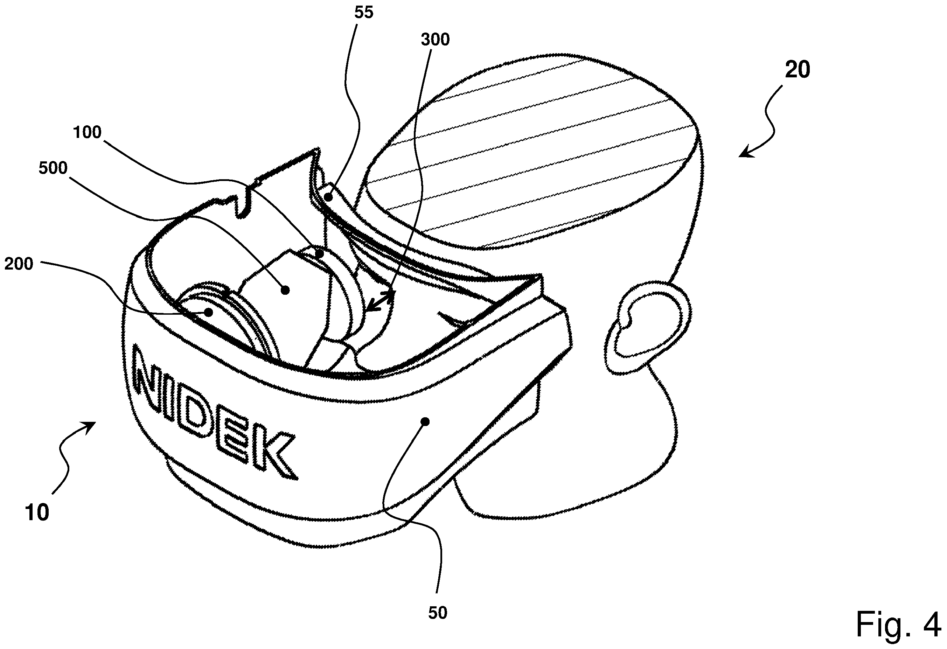

Fig. 1 shows an embodiment of an examination apparatus 10 according to the present invention. In Fig. 1, examination apparatus 10 is associated with a patient 20 to be examined, but the position of apparatus 10 with respect to patient 20 does not correspond exactly to an operating condition; in fact, its mechanical assembly 1000, i.e. the internal frame and the external casing, is not in touch with the face of the patient but is distant about 1 cm (the same is true for Fig. 2 and Fig. 4). In general, the mechanical assembly is arranged to contact a front side of a face of a patient under examination when the examination apparatus is in use; according to the embodiment of Fig. 1, mechanical assembly 1000 is designed to contact at the top forehead 21 and at the bottom upper lip 22 (i.e. the region where the philtrum is located) and/or cheeks 23 on both sides of nose 24.

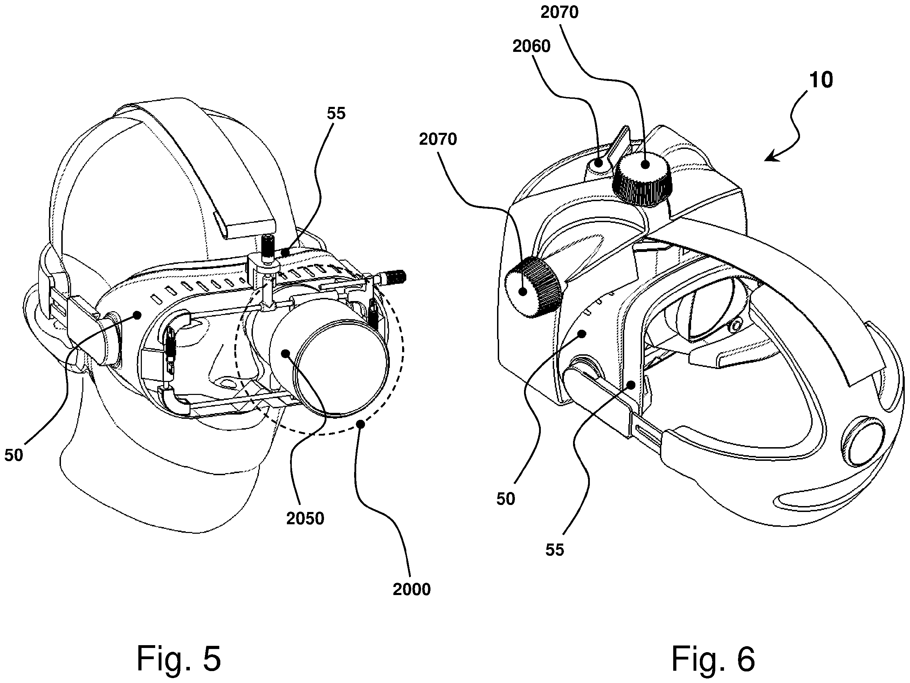

According to the embodiment of Fig. 1, the examination apparatus is head-mounted and the mechanical assembly 1000 is designed to contact not only a front side of a head of a patient, but also a top side and a back side (see e.g. Fig. 5 and Fig. 6). According to alternative embodiments, the examination apparatus may be resting on a table or on a floor through e.g. a base.

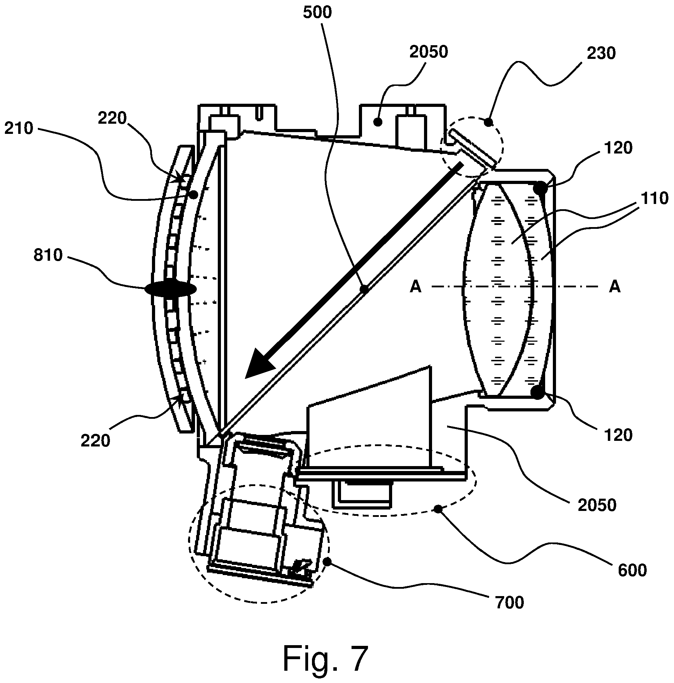

The apparatus according to the present invention has two basic components: an optical assembly (labelled 100 in Fig. 1) and a dome device (labelled 200 in Fig. 1) for the visual field perimetry capabilities; typically and advantageously, there is only one optical assembly and only dome device. In Fig. 1, a mirror 500 is evident and a display 600 is omitted (but see Fig. 7) as the explanation of the blind spot capabilities follows. It is to be noted that the mirror is highly advantageous.

Optical assembly 100 is fixed-focus (i.e. there are no auto-focus capabilities) and mounted (directly or indirectly) to mechanical assembly 1000; it is arranged to be located opposite to eye 25 of patient 20 under examination when examination apparatus 10 is in use.

Dome device 200 is fixed-position and is mounted (directly or indirectly) to mechanical assembly 1000. Dome device 200 has a curved surface 210; for the sake of simplicity, curved surface 210 may be a spherical surface (more accurate shapes may be considered). Dome device 200 is associated to a plurality of light emitting devices 220 (only two devices are associated to the reference numeral in Fig. 7 even if there are many more), for example micro-LEDs, that may be arranged similarly to a traditional "Goldmann perimeter"; specifically, light emitting devices 220 are arranged so to emit light from curved surface 210 towards optical assembly 100; according to an embodiment, curved surface 210 belongs to a curved plate having a plurality of holes wherein a corresponding plurality of light emitting devices are inserted typically from the back of the curved plate. Dome device 200 is arranged to be located behind optical assembly 100 at a fixed position (therefore also at a fixed distance) therefrom and coaxial thereto when examination apparatus 10 is in use.

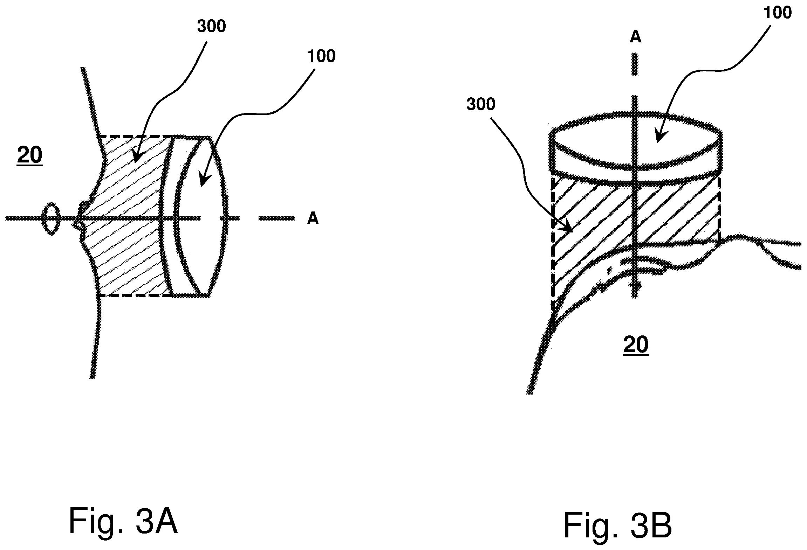

A front side of examination apparatus 10 is configured so that, when it is in use, a volume 300, that may be called "front volume", is defined between the front side of the examination apparatus and the front side of a face of patient 20 under examination sufficient for housing spectacles of patient 20 (see in particular Fig. 3A and Fig. 3B).

Such examination apparatus is very compact and light; in fact, it may take the form of a head-mounted equipment (see the figures).

Thanks to the curvature of the curved surface of the dome device, the optical assembly may be very simple, for example it may correspond to a single lens or more preferably to a doublet (see in particular Fig. 7), still providing accurate examination results.

Thanks to the "front volume" suitable for accommodating spectacles, there is no need for auto-focus capabilities in the examination apparatus still providing accurate examination results.

As it is apparent from the figures, this embodiment of the examination apparatus according to the present invention looks similar to a diving mask or, even more, to a virtual reality goggle, even if it has nothing to do with such apparatuses. There is a mechanical assembly 1000 with a closed shell 50; mechanical assembly 1000 is arranged to be located in front of the eyes 25R and 25L of a patient to be examined; shell 50 is hollow and houses typically only one integrated assembly 2000 (see in particular Fig. 5). Shell 50 has a front portion designed to contact a front side of a face of a patient. Preferably, shell 50 is associated with a coupling element 55 (that may be made of a rigid or flexible material) surrounding the front portion of shell 50 and designed to avoid (or at least limit) that ambient light reaches the eyes of the patent under examination; coupling element 55 may be designed to contact or at least get very close to front side of a face of a patient. Mechanical assembly 1000 comprises a belt or band to secure examination assembly 10 to the head of a patient to be examined; in particular, the belt or band contacts the back of the head (see Fig. 5 and Fig. 6). Another belt or band may be provided contacting the top of the head (see Fig. 5 and Fig. 6).

According to the embodiment of Fig. 1, the examination apparatus is head-mounted and the mechanical assembly is designed to contact not only a front side of a head of a patient, but also a top side and a back side (see e.g. Fig. 5 and Fig. 6).

It is to be noted that the head-mounted feature shown in all attached figures is only a possibility for the present invention; according to alternative embodiments, the examination apparatus may be resting on a table or on a floor through e.g. a base.

The use and operation of an examination apparatus according to the present invention is similar to a traditional "Goldmann perimeter". For example, light emitting devices 220 may be driven accordingly through an appropriate electric and electronic circuitry; in the embodiment of Fig. 1, a first electric and electronic circuitry is integrated inside examination apparatus 10 and a second electric and electronic circuitry is integrated inside an external computer (not shown in the figures) electrically connected to examination apparatus 10. Furthermore, examination apparatus 10 may comprise an electric push-button (not shown in the figures) for patient feedback that may be electrically connected to the first electric and electronic circuitry so that feedback from the patient may be transferred to external computer. According to some embodiments, both the electric and electronic circuitry and the electric push-button are parts of the examination apparatus.

From Fig. 1, it is evident that a frustoconical (or approximately frustoconical) volume 400, that may be called "internal volume", is defined between optical assembly 100 at a first end thereof (close to the eye) and dome device 200 at a second end thereof (remote from the eye); such volume is used for transferring light signals typically from dome device 200 to optical assembly 100 and then through optical assembly 100 to eye 25 of patient 20 under examination. Alternatively, volume 400 may be cylindrical (or approximately cylindrical). Optical assembly 100 and dome device 200 are preferably parts of a same integrated assembly; an embodiment of such assembly is shown in Fig. 7 and labelled 2000. Assembly 2000 comprises a hollow assembly shell 2050 used for housing and mounting several components including optical assembly 100 and dome device 200. In turn, examination apparatus 10 comprises a hollow apparatus shell 50 for housing and mounting at least one assembly shell 2050.

Advantageously, examination apparatus 10 comprises only one integrated assembly 2000; in this way, weight is reduced, cost is reduced and the integrated assembly may be a bit bigger (as there is not another integrated assembly adjacent thereto). In this case, it is preferable that integrated assembly 2000 may be relocated so to be opposite to a right eye 25R or to a left eye 25L of a patient 20 under examination; Fig. 6 shows a lever 2060 (to be acted upon manually) for relocating integrated assembly 2000 between eyes.

Highly advantageously (but not strictly necessarily), a transversal position of optical assembly 100 (i.e. transversal to the optical axis of optical assembly 100) may be adjusted so to align the assembly with pupil 26 of eye 25 of a patient 20 under examination. According to the embodiment of the figures, first examination apparatus 10 is mounted on the head of a patient 20 to be examined, and then such adjustment is made. Fig. 6 shows two knobs 2070 (to be acted upon manually) for carrying out adjustments respectively according to a first axis and to a second axis, the first axis and the second axis being perpendicular to each other and both perpendicular to the optical axis of optical assembly 100. According to the embodiment of the figures, the position of the whole integrated assembly 2000 may be adjusted; in fact, by moving integrated assembly 2000 also optical assembly 100 is correspondingly moved.

Instead, the axial position of optical assembly 100 may be not adjusted; in other words, the solution according to the present invention as set out above does not require an axial adjustment of optical assembly 100. Specifically, with reference to the embodiment of the figures, the solution according to the present invention as set out above does not require an axial adjustment of integrated assembly 2000. Furthermore, considering integrated assembly 2000, the relative position of optical assembly 100 and dome device 200 is fixed and not adjustable.

Examination apparatus 10 comprises an illumination device 230 arranged to illuminate curved surface 210 from a distance in a uniform manner. Such illumination device is typically a single LED; in order to obtain a uniform illumination with a single LED on a curved surface, the LED is not directed to the center of the curved surface but to a point located in a boundary region of the curved surface (see arrow in Fig. 7). By controlling the quantity of light emitted from illumination device 230 it is possible to set in a precise manner the contrast of the image presented to the eye of the patient under examination. It is to be noted that the illumination device is highly advantageous but not strictly necessary for the present invention.

Preferably, illumination device 230 is located outside of internal volume 400 so to that light signals transferred from dome device 200 to optical assembly 100 do not hit illumination device 230. As can be seen in particular in Fig. 7, illumination device 230 is located laterally to internal volume 400; in particular, when examination apparatus 10 is in use, illumination device 230 is located above volume 400.

Advantageously, optical assembly 100 is very simple and, in particular, comprises a lens 110 being a doublet (see Fig. 7) in order to compensate aberrations; it is to be noted that, in the field of optics, the term "lens" is often used to indicate a small set of associated lenses. According to typical and advantageous embodiments of the present invention, the examination apparatus according to the present invention is arranged so that a distance between a front surface of optical assembly 100 and pupil 26 is in the range between 20 mm and 40 mm, preferably about 30 mm; in this way, the apparatus has a very compact size while at the same time this distance is sufficient for housing spectacles of a patient 20.

According to typical and advantageous embodiments of the present invention, curved surface 210 has a circular perimeter and has a diameter in the range between 55 mm and 80 mm, preferably about 65 mm; in this way, the apparatus has a very compact size.

According to a first set of typical and advantageous embodiments of the present invention, a distance between a lens 110 of optical assembly 100 and curved surface 210 corresponds (about) to a back focal length of a lens of optical assembly 100. In this case, a distance between a rear surface of optical assembly 100 and curved surface 210 is advantageously in the range between 55 mm and 75 mm, preferably about 65 mm; in this way, the apparatus has a very compact size. According to a second set of typical and advantageous embodiments of the present invention, a distance between a lens 110 of optical assembly 100 and curved surface 210 is such as to create an image of curved surface 210 in the eye 25 of a patient 20 under examination as if curved surface 210 would be at (about) 30 cm from eye 25. In this case, a distance between a rear surface of optical assembly 100 and curved surface 210 is advantageously in the range between 45 mm and 65 mm, preferably about 55 mm; in this way, the apparatus has a very compact size.

According to typical and advantageous embodiments of the present invention, optical assembly 100 comprises a lens 110 having a focal distance in the range between 60 mm and 90 mm.

According to advantageous embodiments of the present invention, examination apparatus 10 comprises a semi-transparent mirror 500 located between optical assembly 100 and dome device 200, specifically inside internal volume 400; light from optical assembly 100 is transmitted and light from dome device 200 is reflected. Mirror 500 is useful for carrying out one or more functions (in particular examinations) additional to visual field perimetry.

In the embodiment of Fig. 7, semi-transparent mirror 500 is fixedly mounted to integrated assembly 2000.

In general, and also in the embodiment of Fig. 7, semi-transparent mirror 500 is preferably arranged so to fully exploit the surface of the lenses of spectacles of a patient 20 under examination; specifically, when the examination apparatus is in use, mirror 500 reflects downwardly light coming from optical assembly 100. Preferably, semi-transparent mirror 500 is inclined of an angle with respect to an axis A of optical assembly 100; this angle is in the range from 30° to 60°, preferably about 45°.

Preferably, semi-transparent mirror 500 has a transmissivity in the range from 70% to 90% (preferably about 80%) and a reflectivity in the range from 30% to 10% (preferably about 20%) in the visible spectrum.

Preferably, semi-transparent mirror 500 has a transmissivity of about 0% and a reflectivity of about 100% in the IR spectrum.

The examination apparatus 10 comprises an electronic display 600; display 600 is preferably an OLED display due to its high darkness when it is switched off; the images generated on this display are directed towards mirror 500, reflected by mirror 500, directed towards optical assembly 100, focused by optical assembly 100, and finally directed towards eye 25. Display 600 is useful for carrying out blind spot examinations and preferably furthermore for providing instructions and/or information to a patient 20 under examination. It is to be noted that the electronic display is advantageous but not strictly necessary for the present invention.

Preferably, electronic display 600 is located outside of internal volume 400 so to that light signals transferred from dome device 200 to optical assembly 100 do not hit electronic display 600. As can be seen in particular in Fig. 7, electronic display 600 is located laterally to internal volume 400; in particular, when examination apparatus 10 is in use, electronic display 600 is located below volume 400.

In the embodiment of Fig. 7, electronic display 600 is fixedly mounted to integrated assembly 2000.

According to advantageous embodiments of the present invention, examination apparatus 10 comprises a camera 700 for gaze tracking specifically outside of internal volume 400; camera 700 typically consists of a CMOS device and a lens; images of eye 25 coming from optical assembly 100 are directed towards mirror 500, reflected by mirror 500, directed towards camera 700. In this way, it is possible to continuously monitor for example the position of pupil 26 of eye 25. Preferably, camera 700 is located outside of internal volume 400 so that light signals transferred from dome device 200 to optical assembly 100 do not hit camera 700. As can be seen in particular in Fig. 7, camera 700 is located laterally to internal volume 400; in particular, when examination apparatus 10 is in use, camera 700 is located below volume 400.

In the embodiment of Fig. 7, camera 700 is fixedly mounted to integrated assembly 2000.

Advantageously, especially in case of "gaze tracking", examination apparatus 10 comprises IR illumination devices 120 (for example IR LEDs) arranged to illuminate pupil 26 of eye 25 of patient 20 under examination; in this case, camera 20 is an IR camera. Devices 120 may be associated to optical assembly 100 and may be arranged around a front lens of optical assembly 100.

According to advantageous and typical embodiments of the present invention, examination apparatus 10 comprises one fixation light source 810 for visual field perimetry examination. Furthermore, examination apparatus 10 may comprise other two fixation light sources for blind spot examination. Typically, such fixation light sources are single LEDs. According to the best position, such fixation light sources are associated to curved surface 210; in particular, each fixation light source may be inserted from the back of curved plate 210 into a corresponding hole of curved plate 210; fixation light source 810 should be in the center of curved plate 210 while fixation light sources for blind spot examination should be respectively a bit (e. g. 1 cm) to the right and bit (e. g. 1 cm) to the left.

## Claims

1. Examination apparatus (10) with visual field perimetry and blind spot capabilities comprising:
- a mechanical assembly (1000) arranged to contact a front side of a face of a patient under examination (20) when the examination apparatus (10) is in use,
- a fixed-focus optical assembly (100) mounted to said mechanical assembly (1000), and arranged to be located opposite to an eye (25) of a patient under examination (20) when the examination apparatus (10) is in use,
- a fixed-position dome device (200) mounted to said mechanical assembly (1000), having a curved surface (210), associated to a plurality of light emitting devices (220), said dome device (200) being arranged to be located behind said optical assembly (100) at a fixed position therefrom and coaxial thereto when the examination apparatus (10) is in use, and
- an illumination device (230) arranged to illuminate said curved surface (210) of said dome device (200) in a uniform manner,
wherein a front side of the examination apparatus (10) is configured so that, when the examination apparatus (10) is in use, a volume (300) is defined between said front side of the examination apparatus (10) and a front side of a face of a patient under examination (20) sufficient for housing spectacles of a patient under examination (20);wherein a transversal position of said optical assembly (100) may be adjusted so to align it with a pupil (26) of an eye (25) of a patient under examination (20); **characterized by**:
- a semi-transparent mirror (500) being located between said optical assembly (100) and said dome device (200) and being arranged to transmit light from said dome device (200) to said optical assembly (100), and
- an electronic display (600) for blind spot examination, being arranged so that images generated by it are directed towards said optical assembly (100), through reflection by said semi-transparent mirror (500).

2. Examination apparatus (10) according to claim 1, wherein said optical assembly (100) and said dome device (200) are parts of a same integrated assembly (2000).

3. Examination apparatus (10) according to claim 2, wherein said integrated assembly (2000) is unique and may be relocated so to be opposite to a right eye (25R) or to a left eye (25L) of a patient under examination (20).

4. Examination apparatus (10) according to claim 1 or 2 or 3, wherein an axial position of said optical assembly (100) or of said integrated assembly (2000) may be not adjusted.

5. Examination apparatus (10) according to any of the preceding claims, wherein said optical assembly (100) comprises a lens (110) being a doublet.

6. Examination apparatus (10) according to any of the preceding claims, being arranged so that a distance between a front surface of said optical assembly (100) and a pupil (26) of an eye (25) of a patient under examination (20) is in the range between 20 mm and 40 mm, preferably about 30 mm.

7. Examination apparatus (10) according to any of the preceding claims, wherein said curved surface (210) has a circular perimeter and has a diameter in the range between 55 mm and 80 mm, preferably about 65 mm.

8. Examination apparatus (10) according to any of the preceding claims from 1 to 7, wherein a distance between a lens (110) of said optical assembly (100) and said curved surface (210) corresponds to a back focal length of a lens of said optical assembly (100).

9. Examination apparatus (10) according to claim 8, wherein a distance between a rear surface of said optical assembly (100) and said curved surface (210) is in the range between 55 mm and 75 mm, preferably about 65 mm.

10. Examination apparatus (10) according to any of the preceding claims from 1 to 7, wherein a distance between a lens (110) of said optical assembly (100) and said curved surface (210) is such as to create an image of said curved surface (210) in the eye (25) of a patient under examination (20) as if said curved surface (210) would be at 30 cm from said eye (25).

11. Examination apparatus (10) according to claim 10, wherein a distance between a rear surface of said optical assembly (100) and said curved surface (210) is in the range between 45 mm and 65 mm, preferably about 55 mm.

12. Examination apparatus (10) according to any of the preceding claims, wherein said optical assembly (100) comprises a lens (110) having a focal distance in the range between 60 mm and 90 mm.

13. Examination apparatus (10) according to any of the preceding claims, further comprising a camera (700) for gaze tracking.

14. Examination apparatus (10) according to any of the preceding claims, further comprising one fixation light source (810) for visual field perimetry examination.

## Patentansprüche

1. Untersuchungsgerät (10) mit Gesichtsfeldperimetrie und Toter-Winkel-Funktionen, umfassend:
- eine mechanische Baugruppe (1000), die so angeordnet ist, dass sie eine Vorderseite eines Gesichts eines zu untersuchenden Patienten (20) berührt, wenn das Untersuchungsgerät (10) in Gebrauch ist,
- eine optische Baugruppe (100) mit festem Fokus, die an der mechanischen Baugruppe (1000) montiert und so angeordnet ist, dass sie sich gegenüber einem Auge (25) eines zu untersuchenden Patienten (20) befindet, wenn das Untersuchungsgerät (10) in Gebrauch ist,
- eine ortsfeste Kuppelvorrichtung (200), die an der mechanischen Baugruppe (1000) montiert ist und eine gekrümmte Oberfläche (210) aufweist, die mit einer Vielzahl von lichtemittierenden Vorrichtungen (220) assoziiert ist, wobei die Kuppelvorrichtung (200) so angeordnet ist, dass sie sich hinter der optischen Baugruppe (100) an einer festen Position davon befindet und koaxial dazu ist, wenn das Untersuchungsgerät (10) in Gebrauch ist, und
- eine Beleuchtungsvorrichtung (230), die angeordnet ist, um die gekrümmte Oberfläche (210) der Kuppelvorrichtung (200) gleichmäßig zu beleuchten,
wobei eine Vorderseite des Untersuchungsgeräts (10) so konfiguriert ist, dass bei Gebrauch des Untersuchungsgeräts (10) ein Volumen (300) zwischen der Vorderseite des Untersuchungsgeräts (10) und einer Vorderseite eines Gesichts eines zu untersuchenden Patienten (20) definiert ist, das ausreicht, um eine Brille eines zu untersuchenden Patienten (20) aufzunehmen; wobei eine Querposition der optischen Baugruppe (100) so eingestellt werden kann, dass sie mit einer Pupille (26) eines Auges (25) eines zu untersuchenden Patienten (20) ausgerichtet wird;
**gekennzeichnet durch**
- einen halbdurchlässigen Spiegel (500), der sich zwischen der optischen Baugruppe (100) und der Kuppelvorrichtung (200) befindet und angeordnet ist, um Licht von der Kuppelvorrichtung (200) zu der optischen Baugruppe (100) zu übertragen, und
- eine elektronische Anzeige (600) zur Untersuchung des toten Winkels, die so angeordnet ist, dass die von ihr erzeugten Bilder durch Reflexion durch den halbdurchlässigen Spiegel (500) auf die optische Baugruppe (100) gerichtet sind.

2. Untersuchungsgerät (10) nach Anspruch 1, wobei die optische Baugruppe (100) und die Kuppelvorrichtung (200) Teile derselben integrierten Baugruppe (2000) sind.

3. Untersuchungsgerät (10) nach Anspruch 2, wobei die integrierte Baugruppe (2000) einzigartig ist und so verlagert werden kann, dass sie einem rechten Auge (25R) oder einem linken Auge (25L) eines zu untersuchenden Patienten (20) gegenüberliegt.

4. Untersuchungsgerät (10) nach Anspruch 1 oder 2 oder 3, wobei eine axiale Position der optischen Baugruppe (100) oder der integrierten Baugruppe (2000) nicht eingestellt werden kann.

5. Untersuchungsgerät (10) nach einem der vorhergehenden Ansprüche, wobei die optische Baugruppe (100) eine Linse (110) umfasst, die ein Dublett ist.

6. Untersuchungsgerät (10) nach einem der vorhergehenden Ansprüche, das so angeordnet ist, dass ein Abstand zwischen einer Vorderoberfläche der optischen Baugruppe (100) und einer Pupille (26) eines Auges (25) eines zu untersuchenden Patienten (20) im Bereich zwischen 20 mm und 40 mm, vorzugsweise etwa 30 mm, liegt.

7. Untersuchungsgerät (10) nach einem der vorhergehenden Ansprüche, wobei die gekrümmte Oberfläche (210) einen kreisförmigen Umfang aufweist und einen Durchmesser im Bereich zwischen 55 mm und 80 mm, vorzugsweise etwa 65 mm, aufweist.

8. Untersuchungsgerät (10) nach einem der vorhergehenden Ansprüche 1 bis 7, wobei ein Abstand zwischen einer Linse (110) der optischen Baugruppe (100) und der gekrümmten Oberfläche (210) einer hinteren Brennweite einer Linse der optischen Baugruppe (100) entspricht.

9. Untersuchungsgerät (10) nach Anspruch 8, wobei ein Abstand zwischen einer Rückoberfläche der optischen Baugruppe (100) und der gekrümmten Oberfläche (210) im Bereich zwischen 55 mm und 75 mm, vorzugsweise etwa 65 mm, liegt.

10. Untersuchungsgerät (10) nach einem der vorhergehenden Ansprüche 1 bis 7, wobei ein Abstand zwischen einer Linse (110) der optischen Baugruppe (100) und der gekrümmten Oberfläche (210) derart ist, dass ein Bild der gekrümmten Oberfläche (210) in dem Auge (25) eines zu untersuchenden Patienten (20) erzeugt wird, als ob die gekrümmte Oberfläche (210) 30 cm von dem Auge (25) entfernt wäre.

11. Untersuchungsgerät (10) nach Anspruch 10, wobei ein Abstand zwischen einer Rückoberfläche der optischen Baugruppe (100) und der gekrümmten Oberfläche (210) im Bereich zwischen 45 mm und 65 mm, vorzugsweise etwa 55 mm, liegt.

12. Untersuchungsgerät (10) nach einem der vorhergehenden Ansprüche, wobei die optische Baugruppe (100) eine Linse (110) mit einer Fokaldistanz im Bereich zwischen 60 mm und 90 mm umfasst.

13. Untersuchungsgerät (10) nach einem der vorhergehenden Ansprüche, ferner umfassend eine Kamera (700) zur Blickverfolgung.

14. Untersuchungsgerät (10) nach einem der vorhergehenden Ansprüche, ferner umfassend eine Fixierlichtquelle (810) zur Gesichtsfeldperimetrieuntersuchung.

## Revendications

1. Appareil d'examen (10) doté de capacités de périmétrie de champ visuel et de tache aveugle, comprenant :
- un ensemble mécanique (1000) conçu pour entrer en contact avec la face avant du visage d'un patient examiné (20) lorsque l'appareil d'examen (10) est en cours d'utilisation,
- un ensemble optique à focale fixe (100) monté sur ledit ensemble mécanique (1000), et conçu pour être placé à l'opposé d'un oeil (25) d'un patient examiné (20) lorsque l'appareil d'examen (10) est en cours d'utilisation,
- un dispositif de dôme à position fixe (200) monté sur ledit ensemble mécanique (1000), ayant une surface incurvée (210), associé à une pluralité de dispositifs électroluminescents (220), ledit dispositif de dôme (200) étant conçu pour être situé derrière ledit ensemble optique (100) à une position fixe à partir de celui-ci et coaxiale à celui-ci lorsque l'appareil d'examen (10) est en cours d'utilisation, et
- un dispositif d'éclairage (230) conçu pour éclairer de manière uniforme ladite surface incurvée (210) dudit dispositif de dôme (200), dans lequel une face avant de l'appareil d'examen (10) est configurée de telle sorte que, lorsque l'appareil d'examen (10) est en cours d'utilisation, un volume (300) est défini entre ladite face avant de l'appareil d'examen (10) et une face avant du visage d'un patient examiné (20), suffisant pour abriter les lunettes d'un patient examiné (20) ; dans lequel une position transversale dudit ensemble optique (100) peut être réglée de manière à l'aligner avec une pupille (26) d'un oeil (25) d'un patient examiné (20) ;
**caractérisé par**
- un miroir semi-transparent (500) situé entre ledit ensemble optique (100) et ledit dispositif de dôme (200) et disposé de manière à transmettre la lumière dudit dispositif de dôme (200) vers ledit ensemble optique (100), et
- un affichage électronique (600) pour l'examen des angles morts, disposé de manière à ce que les images qu'il génère soient dirigées vers ledit ensemble optique (100), par réflexion sur ledit miroir semi-transparent (500).

2. Appareil d'examen (10) selon la revendication 1, dans lequel ledit ensemble optique (100) et ledit dispositif de dôme (200) font partie d'un même ensemble intégré (2000).

3. Appareil d'examen (10) selon la revendication 2, dans lequel ledit ensemble intégré (2000) est unique et peut être déplacé de manière à être opposé à l'œil droit (25R) ou à l'oeil gauche (25L) d'un patient examiné (20).

4. Appareil d'examen (10) selon la revendication 1, 2 ou 3, dans lequel la position axiale dudit ensemble optique (100) ou dudit ensemble intégré (2000) peut ne pas être réglée.

5. Appareil d'examen (10) selon l'une quelconque des revendications précédentes, dans lequel ledit ensemble optique (100) comprend une lentille (110) qui est un doublet.

6. Appareil d'examen (10) selon l'une quelconque des revendications précédentes, disposé de telle sorte qu'une distance entre une surface avant dudit ensemble optique (100) et une pupille (26) d'un oeil (25) d'un patient examiné (20) soit comprise entre 20 mm et 40 mm, de préférence environ 30 mm.

7. Appareil d'examen (10) selon l'une quelconque des revendications précédentes, dans lequel ladite surface incurvée (210) a un périmètre circulaire et un diamètre compris entre 55 mm et 80 mm, de préférence environ 65 mm.

8. Appareil d'examen (10) selon l'une quelconque des revendications précédentes de 1 à 7, dans lequel une distance entre une lentille (110) dudit ensemble optique (100) et ladite surface incurvée (210) correspond à une distance focale arrière d'une lentille dudit ensemble optique (100).

9. Appareil d'examen (10) selon la revendication 8, dans lequel une distance entre une surface arrière dudit ensemble optique (100) et ladite surface incurvée (210) est comprise entre 55 mm et 75 mm, de préférence environ 65 mm.

10. Appareil d'examen (10) selon l'une quelconque des revendications précédentes de 1 à 7, dans lequel une distance entre une lentille (110) dudit ensemble optique (100) et ladite surface incurvée (210) est telle qu'elle crée une image de ladite surface incurvée (210) dans l'œil (25) d'un patient examiné (20) comme si ladite surface incurvée (210) se trouvait à 30 cm dudit oeil (25).

11. Appareil d'examen (10) selon la revendication 10, dans lequel une distance entre une surface arrière dudit ensemble optique (100) et ladite surface incurvée (210) est comprise entre 45 mm et 65 mm, de préférence environ 55 mm.

12. Appareil d'examen (10) selon l'une quelconque des revendications précédentes, dans lequel ledit ensemble optique (100) comprend une lentille (110) ayant une distance focale comprise entre 60 mm et 90 mm.

13. Appareil d'examen (10) selon l'une quelconque des revendications précédentes, comprenant en outre une caméra (700) pour le suivi du regard.

14. Appareil d'examen (10) selon l'une quelconque des revendications précédentes, comprenant en outre une source lumineuse de fixation (810) pour l'examen périmétrique du champ visuel.
